# EUROPEAN PATENT SPECIFICATION

(11) **EP 0 440 101 B1**
(45) Date of publication and mention of the grant of the patent: **01.05.1996**
(21) Application number: 91100957.9
(22) Date of filing: 25.01.1991
(51) Int. Cl.: A61M 39/00, A61M 1/00, F16K 7/06

(54) **Diverter for the flow of liquid in a circuit**
Stromteiler für eine Flüssigkeit in einem Kreislauf
Repartiteur du débit d'un liquide dans un circuit

(30) Priority: 30.01.1990 IT 1918790
(43) Date of publication of application: 07.08.1991
(73) Proprietor: DIDECO S.p.A., I-41037 Mirandola (province of Modena) (IT)
(72) Inventor: Vescovini, Pietro, I-41036 Medolla (Province of Modena) (IT)
(74) Representative: Modiano, Guido, Dr.-Ing.

(56) References cited:
- DE-A- 1 903 367
- US-A- 4 596 547
- US-A- 4 878 894

## Description

The present invention relates to a diverter for the flow of liquid in a circuit.

It is known that in many circuits which convey liquids, existing in the most varied fields of technology, it is necessary to create, during operation, different conditions for the circulation of said liquid in order to meet different functional requirements.

A typical situation is that which occurs in medical technology, in which circuits exist which are adapted to be connected to a patient for the infusion or extraction of liquid and comprise an extraction pump and a delivery pump for said liquid drawn from a tank; this is the case, for example, of circuits for the infusion or extraction of blood or physiological solution in the left ventricle of a patient during heart surgery.

In such circuits, the extraction and delivery pumps are always in motion, and it is thus the task of the operator to act on the circuit so as to alternately cause the infusion of liquid or the extraction thereof depending on the requirements.

One of the methods adopted by operators according to the prior art in order to act on the circuit is to clamp, by means of conventional forceps, the lines which must be occluded in order to determine the required operating step, but obviously this method does not allow the promptness of intervention which is often required.

Flow diverters have also been proposed based on the presence of a movable element which is in contact with the liquid and is provided with seals defining paths for said liquid within the circuit and capable of alternately providing the required operating steps, but such diverters are not free from the typical drawbacks related to the presence of liquid seals.

Known from DE-A-1 903 367 is a device for aspiration and washing during operations, preferably of the ear, comprising an aspiration tube and a washing tube inserted in a structure for supporting and fixing means for said tubes and means for selectively occluding the two tubes.

US-A-4 878 894 discloses a gas/saline valve, for use in a gas/saline insufflation system, which includes a gas insufflator and a saline source, a first tubing for coupling the gas insufflator to the gas inlet of an arthroscope and a second tubing for coupling of the saline source to the saline inlet of an arthroscope, a housing for the first and second tubings, and a gas/saline control knob having a solid cylinder which has a first and a second semi-cylindrical notches able to selectively compress said tubings thereby closing off or turning on the flow of gas or saline, respectfully, therethrough.

What has been described above for the medical field is also true for other fields of technology, and thus the aim of the present invention is to provide a diverter for the flow of liquid in a circuit which offers maximum speed in actuation and simultaneously ensures the absence of leaks in the circuit from the paths selectively followed by the liquid.

The proposed aim is achieved by a diverter for the flow of liquid in a circuit according to the invention as disclosed in claim 1.

Further characteristics and advantages will become apparent from the description of a preferred but not exclusive embodiment of the invention, illustrated only by way of non-limitative example in the accompanying drawings, wherein:
figure 1 is an exploded view of the diverter according to the invention;
figure 2 is a view of the diverter in the condition of infusion of liquid to the patient;
figure 3 is a schematic view of the plurality of ducts in the functional condition of figure 2;
figure 4 is a view of the diverter in the condition of extraction of liquid from the patient, with simultaneous recirculation of liquid arriving from the delivery pump;
figure 5 is a schematic view of the plurality of ducts in the functional condition of figure 4.

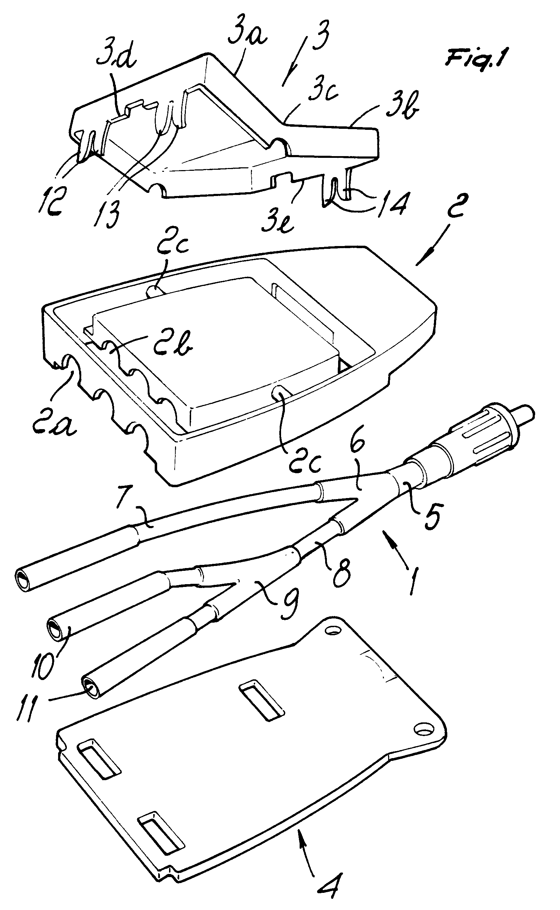

With reference to the above figures, and in particular to figure 1, it can be immediately seen that the diverter comprises a plurality of ducts made of deformable material, generally indicated by the reference numeral 1, inserted in a structure generally indicated by 2 which supports the lid generally indicated by 3 and has a bottom 4.

Considering the individual elements, it can be seen that the plurality of ducts 1 comprises a duct 5 adapted to be connected to a tube, for coupling to the patient, which is intended to convey, for example, blood or physiological solution to the left ventricle during heart surgery.

Said duct 5 has a first branching 6, and the branch 7 is intended to be connected to the tube for coupling to a pump for extracting liquid from the patient, while the other branch has, after a portion 8, a second branching 9 into two branches.

The branch 10 is intended to be connected to the tube for coupling to a pump for the delivery of the liquid from the tank, and the branch 11 is intended to be connected to the tube for the recirculation of the liquid to the tank.

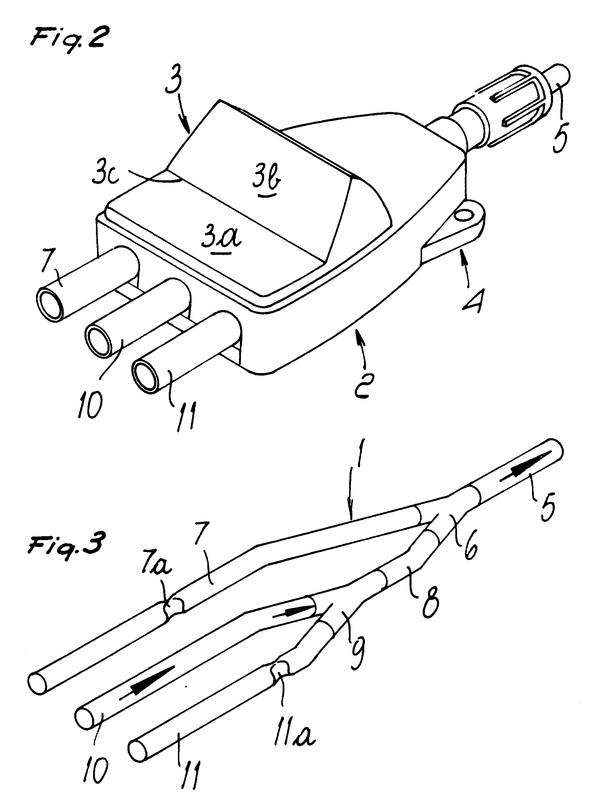

The plurality of ducts 1 is inserted and appropriately retained in place in adapted fixtures such as 2a, 2b in the structure 2 supporting the lid 3 which is pivoted on the axis 2c. Said lid, which for the sake of convenience in manual actuation is shaped according to two planes 3a, 3b which converge at the edge 3c at the pivoting axis, comprises, at the side 3d which is substantially parallel to said pivoting axis, two brackets 12, 13 adapted to simultaneously compress the two branches 7 and 11 until they collapse and thus occlude, as indicated by 7a, 11a in figure 3, at a stroke limit position of the lid.

At the side 3e opposite to the side 3d with respect to the pivoting axis, the lid 3 comprises the bracket 14 adapted to compress the portion 8 until it collapses and occludes as indicated by 8a in figure 5 at the other stroke limit position of the lid.

When liquid is to be infused to the patient, the operator presses the plane 3a of the lid 3 until it moves to the stroke limit position illustrated in figure 2, with occlusion of the ducts 7 and 11 and exclusive opening of the duct 10 which conveys the liquid sent by the delivery pump, which is always in motion, passing through the branching 9, the portion 8 and the branching 6, to the duct 5 connected to the tube for coupling to the patient, as indicated by the arrows of figure 3.

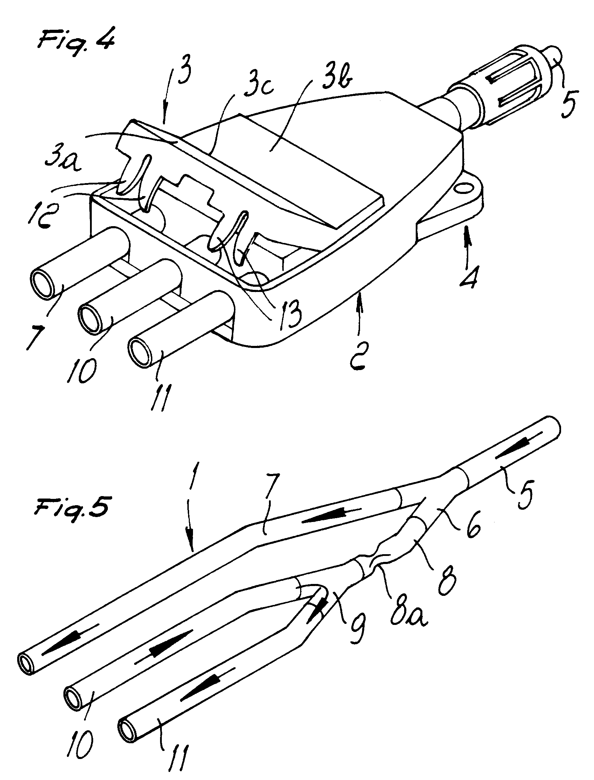

When liquid is to be extracted from the patient with simultaneous recirculation to the tank of the liquid sent by the delivery pump which, as mentioned, is always in motion, the operator presses the plane 3b until it moves to the stroke limit position indicated in figure 4; in this position the brackets 12 and 13 are disengaged from the ducts 7 and 11 which are thus open like the duct 10, while the portion 8 is instead occluded, so that the liquid sent to the duct 10 by the delivery pump recirculates in 11, and the liquid is extracted from the patient through the duct 7, as indicated by the arrows of figure 5.

From the foregoing it is evident that the device according to the invention offers, with the absence of seals in contact with the liquid, the maximum assurance of efficiency in the correct definition of the paths which must alternately be followed by the liquid within the circuit, and the great promptness and speed of intervention allowed to the operator by the device are furthermore evident.

The described invention is susceptible to numerous modifications and variations, all of which are within the scope of the inventive concept; all the details may furthermore be replaced with other technically equivalent elements.

In the practical embodiment of the invention, the materials employed, as well as the shapes and dimensions, may be any according to the requirements.

Where technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

## Claims

1. Diverter for the flow of liquid in a medical circuit, said circuit being adapted to be connected to a patient, and comprising an extraction pump and a delivery pump, said diverter comprising a plurality of ducts (1) made of deformable material, wherein a duct (5) is adapted to be connected to a tube for coupling to the patient, the duct (5) having a first branching (6) which divides into a first (7) and a second (8) branches, said first branch (7) being adapted to be connected to a tube for coupling to the extraction pump, said plurality of ducts (1) being inserted in a supporting structure (2) which comprises a means to selectively compress and occlude said first (7) and second (8) branches, said diverter being characterized in that said second branch (8) has, after a portion (8) of appropriate length, a second branching (9) which divides into a third (10) and a forth (11) branches adapted to be respectively connected to a tube for coupling to the pump for delivery from a tank and to a tube for recirculation to said tank, said means to selectively compress and occlude first (7) and second (8) branches being adapted to occlude, in a first position, the branch portion (8a) comprised between said first and second branchings (6, 9) and, in a second position, the first branch (7a) adapted to be connected to the tube for coupling to the extraction pump and simultaneously with the first branch (7a), the forth branch (11a) adapted to be connected to the recirculation tube.

2. Diverter according to claim 1, characterized in that the means to selectively compress and occlude the branches comprises a manually actuated lid (3) pivoted at a median portion to the supporting structure (2), the lid (3) comprising, at a first side (3e) substantially parallel to the pivoting axis (2c), a bracket (14) adapted to compress, until it collapses at a stroke limit position of the lid (3), the portion (8a) comprised between the first and second branchings (6, 9) of the ducts (1), and two brackets (12,13) at a second side (3d) opposite to the first side (3e) with respect to the pivoting axis (2c), said brackets (12,13) being adapted to simultaneously compress, until they collapse, at the other stroke limit position of the lid (3), the first branch (7) adapted to be connected to the tube for coupling to the extraction pump and the forth branch (11) adapted to be connected to the recirculation tube.

3. Diverter according to one or more of the preceding claims, characterized in that said lid (3) comprises two planes (3a,3b) converging at an edge (3c) whereat said lid (3) is pivoted onto the supporting structure (2).

## Patentansprüche

1. Umlenker für den Strom von Flüssigkeit in einer medizinischen Kreislaufführung, wobei die Kreislaufführung mit einem Patienten verbunden werden kann und eine Extraktionspumpe und eine Zuführpumpe umfaßt, wobei der Umlenker eine Vielzahl von Kanälen (1) umfaßt, die aus einem deformierbren Material hergestellt sind, wobei ein Kanal (5) mit einem Schlauch zum Anschluß an den Patienten verbunden werden kann, wobei der Kanal (5) eine erste Verzweigung (6) aufweist, die sich in einen ersten (7) und einen zweiten (8) Zweig unterteilt, wobei der erste Zweig (7) mit einem Schlauch zum Anschluß an die Extraktionspumpe verbunden werden kann, wobei die Vielzahl von Kanälen (1) in eine Abstützstruktur (2) eingesetzt wird, die ein Mittel umfaßt, um den ersten (7) und den zweiten (8) Zweig selektiv zusammenzudrücken und zu verschließen, wobei der Umlenker dadurch gekennzeichnet ist, daß der zweite Zweig (8) nach einem Bereich (8) einer geeigneten Länge eine zweite Verzweigung (9) aufweist, die sich in einen dritten (10) und einen vierten (11) Zweig unterteilt, die jeweils mit einem Schlauch für den Anschluß an die Pumpe zum Zuführen aus einem Tank und mit einem Schlauch zum Zurückführen zu dem Tank verbunden werden können, wobei das Mittel zum selektiven Zusammenpressen und Verschließen des ersten (7) und des zweiten (8) Zweigs in einer ersten Stellung den Zweigbereich (8a) verschließen kann, der zwischen der ersten und der zweiten Verzweigung (6, 9) enthalten ist und in einer zweiten Stellung den ersten Zweig (7a), der mit dem Schlauch zum Anschluß an die Extraktionspumpe verbunden werden kann, und gleichzeitig mit dem ersten (Zweig 7a) den vierten Zweig (11a), der mit dem Rückführschlauch verbunden werden kann, verschließen kann.

2. Umlenker nach Anspruch 1, dadurch gekennzeichnet, daß das Mittel zum selektiven Zusammendrücken und Verschließen der Zweige einen manuell betätigten Deckel (3) umfaßt, der an einem mittleren Bereich der Abstützstruktur (2) schwenkbeweglich gelagert ist, wobei der Deckel (3) an einer ersten Seite (3e), die im wesentlichen parallel zu der Schwenkachse (2c) ist, einen Bügel (14), der den Bereich (8a), der zwischen der ersten und der zweiten Verzweigung (6, 9) der Kanäle (1) liegt, zusammendrücken kann, bis dieser in einer Hubendstellung des Deckels (3) zusammenfällt, und zwei Bügel (12, 13) an einer zweiten Seite (3d) gegenüber der ersten Seite (3e) mit Bezug auf die Schwenkachse (2c) umfaßt, wobei die Bügel (12, 13) gleichzeitig den ersten Zweig (7), der mit dem Schlauch zum Anschluß an die Extraktionspumpe verbunden werden kann und den vierten Zweig (11), der mit dem Rückführschlauch verbunden werden kann in der anderen Hubendstellung des Deckels (3) zusammendrücken können, bis sie zusammenfallen.

3. Umlenker nach einem der vorhergehenden Ansprüche, dadurch gekennzeichnet, daß der Deckel (3) zwei Ebenen (3a, 3b) aufweist, die an einem Rand (3c) zusammenlaufen, an dem der Deckel (3) an der Abstützstruktur (2) schwenkbeweglich gelagert ist.

## Revendications

1. Répartiteur pour l'écoulement de liquide dans un circuit médical, ledit circuit étant adapté pour être relié à un patient et comprenant une pompe d'extraction et une pompe de refoulement, ledit répartiteur comprenant une pluralité de conduits (1) réalisés en matière déformable, dans lequel un conduit (5) est adapté pour être relié à un tube pour la liaison au patient, le conduit (5) ayant un premier embranchement (6) qui se divise en une première (7) et une deuxième (8) branches, ladite première branche (7) étant adaptée pour être reliée à un tube pour la liaison à la pompe d'extraction, ladite pluralité de conduits (1) étant insérés dans une structure de support (2) qui comprend des moyens pour comprimer et occlure sélectivement lesdites première (7) et deuxième (8) branches,
ledit répartiteur étant caractérisé en ce que ladite deuxième branche (8) présente, après une portion (8) de longueur appropriée, un second embranchement (9) qui se divise en une troisième (10) et une quatrième (11) branches adaptées pour être reliées respectivement à un tube pour la liaison à la pompe pour le refoulement à partir d'un réservoir, et à un tube pour la recirculation vers ledit réservoir, lesdits moyens pour comprimer et occlure sélectivement les première (7) et deuxième (8) branches étant adaptés pour obturer, dans une première position, la portion de branche (8a) comprise entre lesdits premier et second embranchements (6,9) et, dans une seconde position, la première branche (7a) adaptée pour être reliée au tube pour la liaison à la pompe d'extraction et, simultanément avec la première branche (7a), la quatrième branche (11a) adaptée pour être reliée au tube de recirculation.

2. Répartiteur selon la revendication 1,
caractérisé en ce que les moyens pour comprimer et occlure sélectivement les branches comprennent un couvercle (3) actionné manuellement, articulé à une partie médiane sur la structure de support (2), le couvercle (3) comprenant, à un premier côté (3e) sensiblement parallèle à l'axe de pivotement (2c), une patte (14) adaptée pour comprimer, jusqu'à ce qu'elle se replie à une position limite de course du couvercle (3), la portion (8a) comprise entre les premier et second embranchements (6,9) des conduits (1), et deux pattes (12,13) à un second côté (3d) opposé au premier côté (3a) par rapport à l'axe de pivotement (2c), lesdites pattes (12,13) étant adaptées pour comprimer simultanément, jusqu'à ce qu'elles se replient à l'autre position limite de course du couvercle (3), la première branche (7) adaptée pour être reliée au tube pour la liaison à la pompe d'extraction et la quatrième branche (11) adaptée pour être reliée au tube de recirculation.

3. Répartiteur selon l'une ou plusieurs des revendications précédentes,
caractérisé en ce que ledit couvercle (3) comprend deux plans (3a,3b) convergeant à un bord (3c) où ledit couvercle (3) est articulé sur la structure de support (2).
